# FASCICULE DE BREVET EUROPEEN

(11) **EP 0 966 233 B1**
(45) Date de publication et mention de la délivrance du brevet: **21.05.2003**
(21) Numéro de dépôt: 98912544.8
(22) Date de dépôt: 25.02.1998
(51) Int. Cl.: A61B 17/70

(54) **BAGUE POUR DISPOSITIF D'OSTEOSYNTHESE A ANGULATION, ET DISPOSITIF D'OSTEOSYNTHESE L'INCORPORANT**
RING FÜR SCHWENKBARE OSTEOSYNTHESEVORRICHTUNG, UND OSTEOSYNTHESEVORRICHTUNG MIT DIESEM RING
RING FOR AN ANGULATION OSTEOSYNTHESIS DEVICE AND OSTEOSYNTHESIS DEVICE INCORPORATING SAME

(30) Priorité: 26.02.1997 FR 9702298
(43) Date de publication de la demande: 29.12.1999
(73) Titulaire: Stryker France S.A., 93290 Tremblay-en-France (FR)
(72) Inventeur: BACCELLI, Christian, F-33650 Saint Médard d'Eyrans (FR)
(74) Mandataire: Le Forestier, Eric
(86) Numéro de dépôt international: FR9800367
(87) Numéro de publication internationale: WO98037824

(56) Documents cités:
- EP-A- 0 487 895
- FR-A- 2 659 546
- FR-A- 2 682 280
- US-A- 5 242 445

## Description

La présente invention a trait d'une façon générale aux implants pour l'ostéosynthèse notamment du rachis.

Elle concerne plus particulièrement une nouvelle bague de type sphérique destinée à permettre une angulation entre deux composants de l'implant avant serrage, ainsi qu'un implant équipé d'une telle bague.

On connaît par le document FR-A-2 659 546 un système d'ostéosynthèse du rachis qui comprend un ensemble de vis pédiculaires comportant chacune un siège sphérique prévu dans une tête dite "diapason" et dans lequel peut être reçue une bague fendue. Cette bague est elle-même traversée par une tige destinée à relier les diverses vis entre elles, et la bague offre une possibilité d'angulation, dans un ou deux plans, entre l'axe de la vis pédiculaire et l'axe de la tige. Une fois ce réglage angulaire effectué, un bouchon fileté vient comprimer la bague et bloquer fermement l'ensemble.

La bague connue est constituée d'une seule pièce, avec une face extérieure sphérique, une face intérieure cylindrique de révolution, et une fente dirigée radialement et s'étendant entre les faces intérieure et extérieure.

Cette fente confère à la bague la défomabilité requise pour que, lorsqu'elle est serrée dans le siège de la vis pédiculaire à l'aide d'un organe fileté approprié, elle puisse s'écraser fermement contre la tige, de manière à assurer, après le réglage angulaire, une immobilisation parfaite de l'ensemble.

Cete bague connue présente toutefois une limitation : en effet, il est nécessaire, lors de la préparation du système d'implant avant ou au cours de la pose, d'enfiler sur la tige, par l'une de ses extrémités, autant de bagues fendues que nécessaire.

En effet, la déformabilité de la bague conférée par sa fente est loin d'être suffisante pour autoriser un écartement temporaire de cette fente permettant l'introduction radiale de la tige dans le passage cylindrique de la bague. Ainsi le praticien tentant de procéder ainsi provoquerait inévitablement une rupture ou une détérioration importante des qualités mécaniques ou de la forme de la bague.

Ainsi l'utilisation de ce genre de bague connue est limitée au cas où la tige est accessible par au moins l'une de ses extrémités.

La présente invention vise à pallier cet inconvénient de l'état de la technique et à proposer une nouvelle bague déformable qui puisse être rapportée sur une tige même lorsque cette dernière ne comporte aucune extrémité libre au niveau de laquelle la bague puisse être enfilée.

Ainsi la présente invention propose une bague d'angulation pour un dispositif d'ostéosynthèse notamment du rachis, du type comprenant une face extérieure essentiellement sphérique et une face intérieure essentiellement cylindrique, destinée à recevoir une tige du dispositif, bague caractérisée en ce qu'elle est réalisée en deux pièces complémentaires, chaque pièce présentant sensiblement la forme d'un "U" avec un fond et au moins une paire de branches occupant une fraction de la dimension axiale du fond, la distance entre les extrémités des deux branches d'une même paire étant voisine du diamètre d'une tige destinée à recevoir la bague, et les deux pièces étant aptes à être insérées sur la tige à partir du côté de celle-ci de telle sorte que leurs fonds respectifs se trouvent sensiblement à l'opposé l'un de l'autre en emprisonnant la tige et que leurs paires de branches se trouvent côte-à-côte, lesdites deux pièces définissant alors conjointement une face extérieure essentiellement sphérique et une face intérieure essentiellement cylindrique.

Des aspects préférés, mais non limitatifs, de la bague selon l'invention sont les suivants :
- chaque pièce possède une paire de branches, et chacune desdites branches occupe sensiblement la moitié de la dimension axiale de la bague.
- chacune desdites branches possède à son extrémité libre une forme saillante apte à être reçue dans un logement essentiellement complémentaire prévu au niveau du fond de l'autre pièce.
- la forme saillante de chacune des branches d'une pièce est définie par l'intersection d'un contour sphérique de la branche avec une face terminale essentiellement parallèle à l'axe de la bague et prolongeant une surface intérieure semi-cylindrique définie par le fond de cette même pièce.
- ladite face terminale de chaque branche s'étend légèrement en oblique par rapport à l'axe de la bague.
- les deux branches sont séparées du fond par deux épaulements s'étendant dans des plans présentant par rapport à l'axe de la bague une obliquité semblable à celle desdites faces terminales.
- les branches de chaque pièce sont délimitées, au niveau d'une face tournée vers les branches de l'autre pièce, par un plan perpendiculaire à l'axe de la bague.
- la distance minimale entre les extrémités des deux branches d'une même paire est localement légèrement inférieure au diamètre d'une tige destinée à recevoir ladite bague.

L'invention propose également un dispositif d'ostéosynthèse notamment du rachis, comprenant une partie de fixation osseuse possédant un siège essentiellement sphérique, une bague logée dans ledit siège, un élément de serrage de la bague dans son siège, et une tige traversant un passage essentiellement cylindrique formé dans la bague, caractérisé en ce que la tige fait partie d'un composant comportant aux deux extrémités de la tige deux parties plus larges que ladite tige, et en ce que la bague est réalisée comme défini ci-dessus, ses deux parties étant mises en place sur la tige à partir du côté de celle-ci.

D'autres aspects, buts et avantages de la présente invention apparaîtront mieux à la lecture de la description détaillée suivante d'une forme de réalisation préférée de celle-ci, donnée à titre d'exemple et faite en référence au dessin annexé, sur lequel :
la figure 1 est une vue dans l'axe d'une partie de bague selon la présente invention,
la figure 2 est une vue de côté selon la flèche II de la figure 1,
la figure 3 est une vue de côté selon la flèche III de la figure 1,
la figure 4 est une vue de dessus selon la flèche IV de la figure 1,
la figure 5 est une vue en perspective de la partie de bague des figures 1 à 4,
la figure 6 est une vue en perspective d'une tige d'ostéosynthèse sur laquelle a été montée une bague constituée par deux parties de bague identiques à celle illustrée sur les figures 1 à 5, et
la figure 7 est une vue en coupe transversale illustrant le montage d'une partie de bague sur une tige d'ostéosynthèse.

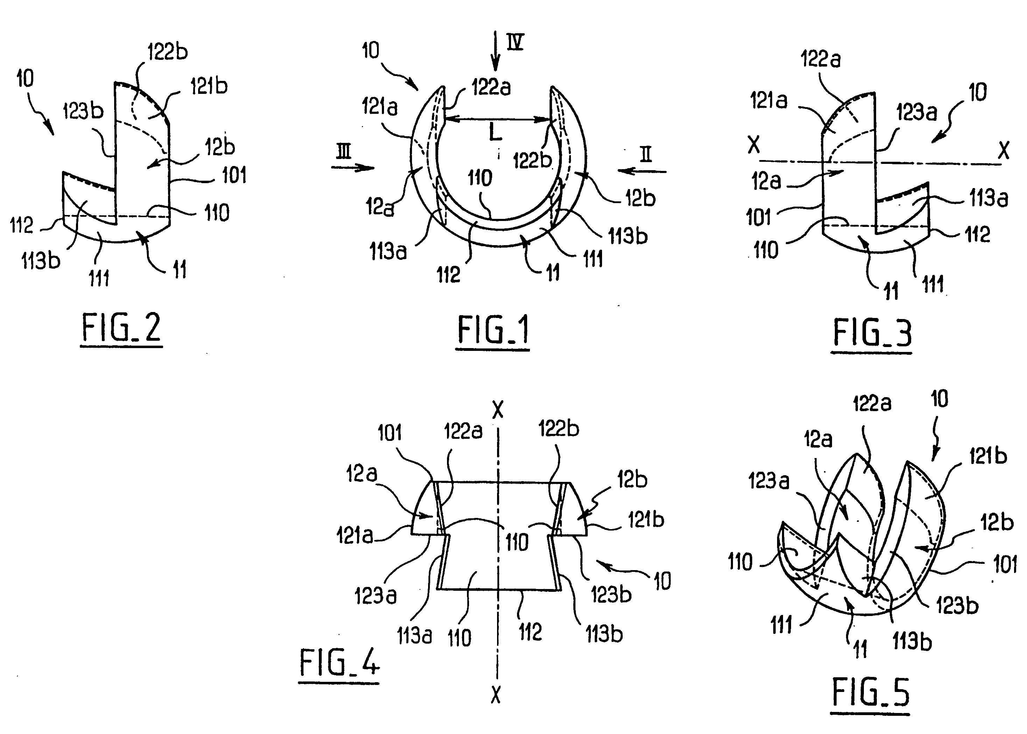

En référence tout d'abord aux figures 1 à 5, on a représenté une pièce destinée à former avec une autre pièce, en l'espèce identique, une bague à surface extérieure essentiellement sphérique et à surface intérieure essentiellement cylindrique.

Cette pièce 10 présente en vue de face (figure 1) la forme générale d'un "U", avec une partie de fond 11 et deux branches latérales 12a, 12b, ces trois parties étant délimitées par des surfaces extérieures respectives 111, 121a, 121b qui appartiennent à une sphère commune.

La partie de fond 11 s'étend, suivant l'axe XX (c'est-à-dire horizontalement sur les figures 2 et 3) une dimension égale à la longueur axiale de la bague, tandis que les deux branches 12a, 12b présentent seulement, dans cette même direction, seulement environ la moitié de cette dimension, le fond et les deux branches présentant une face terminale commune 101 s'étendant perpendiculairement à l'axe XX de la bague.

La transition entre le fond et les branches s'effectue, selon l'axe XX, par deux épaulements ou pans coupés 113a, 113b qui, comme le montre en particulier la figure 4, s'étendent dans des plans légèrement rentrants de l'extérieur vers l'intérieur de la bague.

Du côté opposé au bord commun 101, le fond est délimité par un bord opposé 112 s'étendant dans un plan perpendiculaire à l'axe XX, tandis que les branches 12a, 12b sont délimités par des bords opposés respectifs 123a, 123b s'étendant dans un autre plan perpendiculaire à l'axe XX est situé sensiblement à mi-chemin des plans limites de la partie de fond.

La partie de fond 11 présente une face intérieure 110 qui est une portion d'un cylindre de révolution et qui se prolonge au niveau des faces intérieures des deux branches 12a, 12b, pour former essentiellement un demi-cylindre.

Dans leur région d'extrémité libre, les faces intérieures des deux branches 12a, 12b présentent un pan coupé, respectivement 122a, 122b, qui s'étend essentiellement dans le prolongement de la paroi semi-cylindrique 111, en présentant toutefois une légère obliquité, à savoir une orientation rentrante de l'extérieur vers l'intérieur. Cette obliquité est de préférence voisine de celle des épaulements 113a, 113b.

La distance minimale L entre les deux branches perpendiculairement à l'axe XX (voir figure 1) est choisie légèrement inférieure au diamètre d'une tige sur laquelle deux pièces 10 doivent être montées pour former ensemble une bague sphérique d'angulation.

On observera ici que cette distance minimale L se situe au niveau des pans coupés 122a, 122b, tandis qu'au-dessous de cet étranglement, la dimension de l'espace intérieur de la pièce perpendiculairement à l'axe XX est supérieure à celle de la tige associée, en choisissant pour la paroi semi-cylindrique 110 un diamètre légèrement supérieur au diamètre de la tige.

A titre d'exemple, et pour une pièce 10 réalisée en un matériau du type alliage de titane, ou d'élasticité comparable, la distance L est de préférence inférieure d'environ 15% au diamètre de la tige. En outre, le diamètre de la surface 110 est suffisamment supérieur au diamètre de la tige pour obtenir la liberté de coulissement nécessaire.

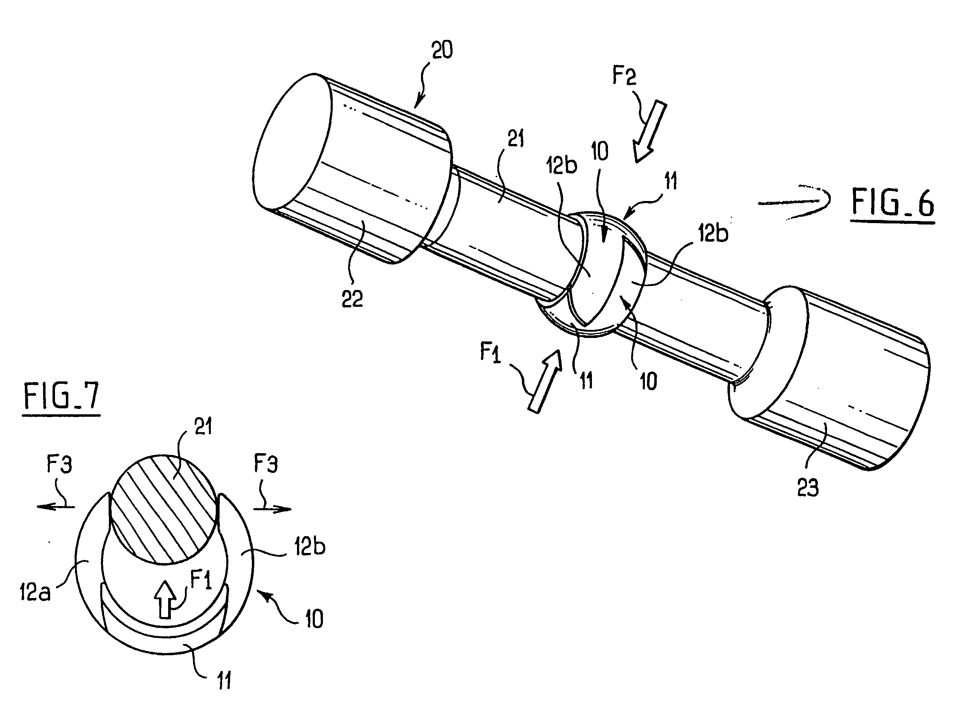

Maintenant en référence aux figures 6 et 7, une bague sphérique est réalisée sur une tige cylindrique 21 en insérant une première pièce 10 sur cette tige, et plus précisément en plaçant cette pièce de telle sorte que son espace intérieur s'ouvre vers la tige et en exerçant un effort radial selon la flèche F1.

Les branches 12a, 12b de la pièce 10 s'écartent alors élastiquement de façon temporaire pour autoriser la pénétration de la tige dans son espace intérieur; après ce franchissement, la tige 21 se trouve reçue, avec un jeu réduit, dans son berceau semi-cylindrique défini par la paroi 110 de la pièce. La pièce 10 peut alors librement coulisser le long de la tige, et emprisonnée sur elle en ce sens que le retrait de la pièce ne peut s'effectuer qu'avec un outil mince, formant levier.

Une seconde pièce 10, identique à la première, est alors placée sur la tige 21 de la même manière, mais en étant orientée dans une position inversée ou tête-bêche par rapport à la pièce déjà mise en place, et en subissant un effort selon la flèche F2, de sens opposé à la flèche F1.

Le positionnement de la seconde pièce par rapport à la première au moment de cette mise en place est choisi de telle sorte que les branches 12a, 12b de la seconde pièce viennent longer les branches 12a, 12b de la première pièce.

On observera ici que la partie terminale de chaque branche, définie par les parties terminales des parois 121a, 122a, 123a (respectivement des parois 121b, 122b, 123b) présente la forme d'une pointe qui, à la fin de la mise en place de la seconde pièce 10, vient se caler dans un logement de forme essentiellement complémentaire défini par d'une part par la surface extérieure de la tige 21 et d'autre part par la face 113a et par la face 123a de la pièce 10 antérieurement mise en place (respectivement par la surface extérieure de la tige 21 et par les faces 113b et 123b). Par symétrie, le même calage est réalisé entre les pointes des branches 12a, 12b de la pièce 10 antérieurement mise en place et les logements correspondants de la pièce nouvellement mise en place.

Ainsi la tige 21 permet d'éviter les translations des deux pièces 10 dans un plan perpendiculaire à son axe, tandis que le quadruple calage précité permet d'assurer que les deux pièces 10 soient étroitement imbriquées l'une dans l'autre, comme le montre la figure 6, sans pouvoir se séparer l'une de l'autre.

Les deux pièces 10 forment ainsi, d'une manière extrêmement simple et facile, une bague sphérique qui n'a pas besoin d'être enfilée à partir d'une extrémité de la tige contrairement au cas d'une bague fendue ordinaire.

Ainsi, la présente invention permet d'équiper une tige 21 d'une bague sphérique notamment lorsque la tige appartient à un élément d'ostéosynthèse, globalement indiqué en 20, où elle forme une réunion entre deux éléments plus larges, indiqués schématiquement en 22, 23 sur la figure 6. Cet élément d'ostéosynthèse 20 peut être notamment une pièce de liaison avec le sacrum.

Une fois la bague mise en place sur la tige 21, cette bague peut coopérer avec les autres composants d'un implant tel que décrit notamment dans le document FR-A-2 659 546, la bague selon l'invention remplaçant la bague fendue d'une seule pièce décrite dans ce document. Plus précisément, la forme en "U' de chaque pièce 10 formant la bague selon l'invention présente la déformabilité élastique requise pour que, lorsque le la bague est comprimée par le serrage du bouchon fileté de l'implant, elle bloque fermement la tige 21 contre toute translation et contre tout basculement par rapport à l'angulation donnée avant serrage. Naturellement, l'invention est également applicable à d'autres types d'implants à bague.

Bien entendu, la présente invention n'est nullement limitée à la forme de réalisation décrite ci-dessus et représentée sur les dessins.

En particulier, on peut prévoir que chacune des pièces 10 comporte plusieurs paires de branches espacées les unes des autres, entre lesquelles viennent s'engager les branches de l'autre pièce.

Par ailleurs, bien que les deux pièces 10 utilisées pour former la bague soient, dans le présent exemple, rigoureusement identiques, ce qui simplifie la fabrication et la manipulation, on peut prévoir deux pièces différentes.

## Revendications

1. Bague d'angulation pour un dispositif d'ostéosynthèse notamment du rachis, du type comprenant une face extérieure essentiellement sphérique et une face intérieure essentiellement cylindrique, destinée à recevoir une tige (21) du dispositif, bague **caractérisée en ce qu'**elle est réalisée en deux pièces complémentaires (10), chaque pièce présentant sensiblement la forme d'un "U" avec un fond (11) et au moins une paire de branches (12a, 12b) occupant une fraction de la dimension axiale du fond, la distance (L) entre les extrémités des deux branches d'une même paire étant voisine du diamètre d'une tige destinée à recevoir la bague, et les deux pièces étant aptes à être insérées sur la tige à partir du côté de celle-ci de telle sorte que leurs fonds respectifs se trouvent sensiblement à l'opposé l'un de l'autre en emprisonnant la tige et que leurs paires de branches se trouvent côte-à-côte, lesdites deux pièces définissant alors conjointement une face extérieure essentiellement sphérique et une face intérieure essentiellement cylindrique.

2. Bague selon la revendication 1, **caractérisée en ce que** chaque pièce (10) possède une paire de branches (12a, 12b) et **en ce que** chacune desdites branches occupe sensiblement la moitié de la dimension axiale de la bague.

3. Bague selon l'une des revendications 1 et 2, **caractérisée en ce que** chacune desdites branches (12a, 12b) possède à son extrémité libre une forme saillante apte à être reçue dans un logement essentiellement complémentaire prévu au niveau du fond de l'autre pièce.

4. Bague selon la revendication 3, **caractérisée en ce que** la forme saillante de chacune des branches (12a, 12b) d'une pièce est définie par l'intersection d'un contour sphérique (121a, 121b) de la branche avec une face terminale (122a, 122b) essentiellement parallèle à l'axe de la bague et prolongeant une surface intérieure semi-cylindrique (110) définie par le fond (11) de cette même pièce.

5. Bague selon la revendication 4, **caractérisé en ce que** ladite face terminale (122a, 122b) de chaque branche s'étend légèrement en oblique par rapport à l'axe (XX) de la bague.

6. Bague selon la revendication 5, **caractérisé en ce que** les deux branches sont séparées du fond par deux épaulements (113a, 113b) s'étendant dans des plans présentant par rapport à l'axe (XX) de la bague une obliquité semblable à celle desdites faces terminales (122a, 122b).

7. Bague selon l'une des revendications 1 à 6, **caractérisé en ce que** les branches (12a, 12b) de chaque pièce sont délimitées, au niveau d'une face tournée vers les branches de l'autre pièce, par un plan (123a, 123b) perpendiculaire à l'axe de la bague.

8. Bague selon l'une des revendications 1 à 7, **caractérisée en ce que** la distance minimale (L) entre les extrémités des deux branches (12a, 12b) d'une même paire est localement légèrement inférieure au diamètre d'une tige (21) destinée à recevoir ladite bague.

9. Dispositif d'ostéosynthèse notamment du rachis, comprenant une partie de fixation osseuse possédant un siège essentiellement sphérique, une bague logée dans ledit siège, un élément de serrage de la bague dans son siège, et une tige (21) traversant un passage essentiellement cylindrique formé dans la bague, **caractérisé en ce que** la tige fait partie d'un élément (20) comportant aux deux extrémités de la tige deux parties (22, 23) faisant obstacle à l'enfilage d'une bague monobloc sur la tige, et **en ce que** la bague est réalisée selon l'une des revendications 1 à 8, ses deux pièces (10, 10) étant mises en place sur la tige à partir du côté de celle-ci.

## Patentansprüche

1. Ring zur Winkelstellung für eine Osteosynthesevorrichtung besonders der Wirbelsäule, der der Art nach eine äußere, im wesentlichen kugelige Fläche und eine innere, im wesentlichen zylindrische Fläche aufweist, die dazu bestimmt ist, eine Stange (21) der Vorrichtung aufzunehmen, wobei der Ring **dadurch gekennzeichnet ist, daß** er aus zwei komplementären Stücken (10) hergestellt ist, wobei jedes Stück im wesentlichen die Form eines "U", mit einem Boden (11) und mindestens einem Paar Armen (12a, 12b) aufweist, die einen Bruchteil der axialen Abmessung des Bodens einnehmen, wobei der Abstand (L) zwischen den Enden der beiden Arme ein und desselben Paares nahe dem Durchmesser einer Stange liegt, die dazu bestimmt ist, den Ring aufzunehmen, und die beiden Stücke dazu eingerichtet sind, auf die Stange von der Seite dieser derart aufgeschoben zu werden, daß ihre jeweiligen Böden sich im wesentlichen einander gegenüberliegend befinden und die Stange einschließen, und daß ihre Paare von Armen sich nebeneinanderliegend befinden, wobei die genannten beiden Stücke dann gemeinsam eine im wesentlichen kugelige Außenfläche und eine im wesentlichen zylindrische Innenfläche definieren.

2. Ring nach Anspruch 1, **dadurch gekennzeichnet, daß** jedes Stück (10) ein Paar Arme (12a, 12b) besitzt, und daß jeder der genannten Arme im wesentlichen die Hälfte der axialen Abmessung des Rings einnimmt.

3. Ring nach einem der Ansprüche 1 oder 2, **dadurch gekennzeichnet, daß** jeder der genannten Arme (12a, 12b) an seinem freien Ende eine vorstehende Form besitzt, die dazu eingerichtet ist, in einem im wesentlichen komplementären Sitz aufgenommen zu werden, der auf Höhe des Bodens des anderen Stücks vorgesehen ist.

4. Ring nach Anspruch 3, **dadurch gekennzeichnet, daß** die vorspringende Form eines jeden der Arme (12a , 12b) eines Stücks definiert ist durch den Schnitt einer kugeligen Kontur (121a, 121b) des Armes mit einer Endfläche (122a, 122b), die im wesentlichen parallel zur Achse des Ringes verläuft und eine halbzylindrische Innenoberfläche (110) verlängert, die durch den Boden (11) dieses selben Stücks gebildet ist.

5. Ring nach Anspruch 4, **dadurch gekennzeichnet, daß** die genannte Endfläche (122a, 122b) eines jeden Armes sich leicht schräg bezüglich der Achse (XX) des Ringes erstreckt.

6. Ring nach Anspruch 5, **dadurch gekennzeichnet, daß** die beiden Arme vom Boden durch zwei Schultern (113a, 113b) getrennt sind, die sich in Ebenen erstrecken, die bezüglich der Achse (XX) des Ringes einen schrägen Verlauf darbieten, der dem der genannten Endflächen (122a, 122b) ähnelt.

7. Ring nach einem der Ansprüche 1 bis 6, **dadurch gekennzeichnet, daß** die Arme (12a, 12b) eines jeden Stückes auf Höhe einer Fläche, die den Armen des anderen Stückes zugewandt ist, durch eine Ebene (123a, 123b) begrenzt sind, die sich senkrecht zur Achse des Ringes erstreckt.

8. Ring nach einem der Ansprüche 1 bis 7, **dadurch gekennzeichnet, daß** der Mindestabstand (L) zwischen den Enden der beiden Arme (12a, 12b) ein und desselben Paares örtlich ein wenig kleiner ist als der Durchmesser einer Stange (21), die dazu bestimmt ist, den genannten Ring aufzunehmen.

9. Osteosynthesevorrichtung besonders der Wirbelsäule, mit einem Teil zur Knochenfestlegung, das einen im wesentlichen kugeligen Sitz aufweist, einen Ring, der im genannten Sitz aufgenommen ist, ein Klemmelement für den Ring in seinem Sitz, und eine Stange (21), die einen im wesentlichen zylindrischen Kanal durchquert, der im Ring ausgebildet ist, **dadurch gekennzeichnet, daß** die Stange Teil eines Elements (20) bildet, das an den beiden Enden der Stange zwei Teile (22, 23) aufweist, die ein Hindernis für das Aufschieben eines einteiligen Ringes auf die Stange bilden, und daß der Ring nach einem der Ansprüche 1 bis 8 ausgebildet ist, wobei seine beiden Stücke (10, 10) von der Seite der Stange her auf ihren Platz auf dieser aufgebracht werden.

## Claims

1. Angular orientation ring for an osteosynthesis device, particularly for the spine, of the type comprising an essentially spherical outer face and an essentially cylindrical inner face intended to take a rod (21) of the device, the ring being **characterized in that** it is made in two mating parts (10), each part being substantially U-shaped with a bottom (11) and at least one pair of branches (12a, 12b) occupying a fraction of the axial dimension of the bottom, the distance (L) between the ends of the two branches of one same pair being close to the diameter of a rod intended to take the ring, and the two parts being able to be inserted over the rod from its side in such a way that their respective bottoms come to substantially face each other, enclosing the rod, and such that their pairs of branches come to be side by side, the said two parts therefore jointly defining an essentially spherical outer face and an essentially cylindrical inner face.

2. Ring according to claim 1, **characterized in that** each part (10) has a pair of branches (12a, 12b) and **in that** each of the said branches occupies substantially half of the axial dimension of the ring.

3. Ring according to one of claims 1 and 2, **characterized in that** each of the said branches (12a, 12b) has, at its free end, a projecting shape able to be received in an essentially complementary housing provided in the bottom of the other part.

4. Ring according to claim 3, **characterized in that** the projecting shape of each of the branches (12a, 12b) of one part is defined by the intersection of a spherical contour (121a, 121b) of the branch with an end face (122a, 122b) essentially parallel to the axis of the ring and forming an extension of a semi-cylindrical inner surface (110) defined by the bottom (11) of this same part.

5. Ring according to claim 4, **characterized in that** the said end face (122a, 122b) of each branch extends slightly obliquely with respect to the axis (XX) of the ring.

6. Ring according to claim 5, **characterized in that** the two branches are separated from the bottom by two shoulders (113a, 113b) extending in planes which, with respect to the axis (XX) of the ring, have an obliqueness similar to that of the said end faces (122a, 122b).

7. Ring according to one of claims 1 to 6, **characterized in that** the branches (12a, 12b) of each part are delimited, on a face facing towards the branches of the other part, by a plane (123a, 123b) perpendicular to the axis of the ring.

8. Ring according to one of claims 1 to 7, **characterized in that** the minimum distance (L) between the ends of the two branches (12a, 12b) of one same pair is locally slightly shorter than the diameter of a rod (21) intended to take the said ring.

9. Osteosynthesis device, particularly for the spine, comprising a bone fixation part which has an essentially spherical seat, a ring housed in the said seat, an element for clamping the ring into its seat, and a rod (21) passing through an essentially cylindrical passage formed in the ring, **characterized in that** the rod forms part of an element (20) comprising, at the two ends of the rod, two parts (22, 23) which constitute an obstacle to the slipping of a one-piece ring over the rod, and **in that** the ring is made according to one of claims 1 to 8, its two parts (10, 10) being fitted onto the rod from the side thereof.
